# EUROPEAN PATENT SPECIFICATION

(11) **EP 1 203 585 B1**
(45) Date of publication and mention of the grant of the patent: **16.03.2005**
(21) Application number: 00937410.9
(22) Date of filing: 08.06.2000
(51) Int. Cl.: A61P 9/10, A61K 31/4015

(54) **ANTI-ISCHEMIC AGENT**
ANTI-ISCHEMISCHER WIRKSTOFF
AGENT ANTI-ISCHEMIQUE

(30) Priority: 21.07.1999 EA 870008
(43) Date of publication of application: 08.05.2002
(73) Proprietor: Zakrytoe Aktsionernoe Obschestvo "ASGL-Issledovatelskie Laboratorii", St. Petersburg, 197198 (RU)
(72) Inventor: TJURENKOV, Ivan Nikolaevich, Volgograd, 400120 (RU); BERESTOVITSKAYA, Valentina Mikhailovna, St.Petersburg, 196070 (RU); ZOBACHEVA, Maya Mikhailovna, St.Petersburg, 198188 (RU); VASILEVA, Olga Sergeevna, St.Petersburg, 195212 (RU); USIK, Nina Vladimirovna, St.Petersburg, 197198 (RU); NOVIKOV, Boris Mikhailovich, St.Petersburg, 196128 (RU)
(74) Representative: Zellentin, Rüdiger, Dr.
(86) International application number: PCT/RU2000/000222
(87) International publication number: WO 2001/005399

(56) References cited:
- EP-A- 0 363 489
- EP-A1- 0 358 008
- FR-A- 2 361 882
- RU-C1- 2 050 851
- D.M. LOMTADZE ET AL.: 'Otsutstvie parallelizma mezhdu antigipoksicheskim i protivoishemicheskim deistviem farmakologicheskikh sredstv' FARMAKOLOGIYA I TOXIKOLOGIYA vol. 54, no. 3, May 1991 - June 1991, XP002953387
- B.S. VILENSKY ET AL.: 'lekarstvennye sredstva dlya differentsirovannoi terapii ostrykh narusheny mozgovogo krovoobrascheniya' PERVY ROSSIISKY NATSIONALNY KONGRESS, (CHELOVEK I LEKARSTVO). TEZISY, 12-16 APRIL, M., ABSTRACT NO. 128. XP002955363

## Description

### Field of technology

The invention relates to medicine and pharmaceutical industry and concerns pharmaceutical remedies for heart disease treatment.

### Previous level of technology

A number of biologically active substances are known, obtaining cardiovascular activity, which differ in structure and character of pharmacological effect.

For example, **2-morpholino-5-phenyl-6H-1,3,4-thiadiazine hydrochloride** [1] has marked inotrop activity, which reduces heart-contracting function. **cis-1-(3-acethylthiopropionyl-6-methyl-pythecomine acid** [2] is used in treatment of coronary deficiency. Well-known is the medicine [3] - **3-methyl-[2-(3-tret.butylamino-2-oxypropoxy) phenoxymethyl]-1,2,4-oxadiazole,** which has marked anti-hypertensive, anti-arrhythmic, antianginal, anti-ischemic and anti-glaucomatous activity. However, anti-ischemic activity of these drugs is unknown or is insufficiently low.

One of the principal groups in pharmacotherapy of ischemic heart disease (the most widely spread disease of cardiovascular system) are β-adrenoblockers. One of the most widely used medicines of β-adrenoblockers group is Obsidan (Propranolol, Anaprylin®) [4]. Anti-ischemic activity of Obsidan is connected with the capacity of the drug to reduce myocardial need for oxygen at the expense of decrease of the heart rate by reducing systole capacity of myocard and extension of the coronary flow. Yet, the use of Obsidan is limited in patients with obstruent lungs diseases due to the broncho-spastic effect and in patients with obliterating diseases of peripheral vessels due to the drug's negative inotrop effect. β-adrenoblockers ability to increase the level of atherogene lipid fractions in blood is known as well.

**4-phenyl-2-pyrrolidinone-1-acetic acid amide** is known as the substance obtaining hypotensive activity [5]. Its nootropic activity has been described [6], however its anti-ischemic activity has not been described yet.

### Disclosure of the invention

The purpose of the invention - to develop an effective anti-ischemic medicine and to extend the range of such drugs.

This purpose is realised by the use of **4-phenyl-2-pyrrolidinone-1-acetic acid amide** as the anti-ischemic medicine.

### Examples of invention realisation

Study of **4-phenyl-2-pyrrolidinone-1-acetic acid amide** anti-ischemic activity.

The study on **4-phenyl-2-pyrrolidinone-1-acetic acid amide** anti-ischemic activity has been carried out in model of experimental myocardial infarction caused by the experimental myocardial infarction after the descending branch of the left coronary artery (LAD) occlusion in rats. **4-phenyl-2-pyrrolidinone-1-acetic acid amide** was administered intraperitoneally in dose 50 mg/kg 30 minutes before the LAD-occlusion. Anti-ischemic activity of the substance was assessed on the third day after its administration by estimation of the area of necrosis, the area of ischemia and the total myocardial necrosis zone (area of necrosis plus area of ischemia). Ischemia zone and myocardial injury zones were assessed by morphometry methods. Results obtained in the experimental animals' groups exposed to the substance were compared with results obtained in animals, exposed to Obsidan and physiological solution (PhS) in dose 0.5 mg/kg, administered 30 minutes before the LAD occlusion.

Study results are presented in the Table 1.

**Table 1.**

| PhP (4-phenyl-2-pyrrolidinone-1-acetic acid amide) and Obsidan influence on the area of necrosis, the area of ischemia and the total myocardial necrosis zone of rats with experimental myocardial infarction. | | | | | | | |
|---|---|---|---|---|---|---|---|
| Group | Area of the myocardial zone (mm ² ) | Area of the ischemia zone (mm ² ) | Ratio: ischemia zone/ myocard | Area of the necrosis zone (mm ² ) | Ratio: necrosis zone /myocard | Area of the injury zone (MM ² ) | Ratio: necrosis/ ischemia |
| Control (PhS) | 485,97± | 233,09± | 0,479± | 36,00± | 0,074± | 269,09± | 0,165± |
| | 4,87 | 11,34 | 0,022 | 5,84 | 0,012 | 9,07 | 0,036 |
| Obsidan | 481,13± | 175,21± | 0,365± | 28,48 | 0,059± | 203,68± | 0,163± |
| | 8,12 | 4,01 * | 0,009 * | ±4,08 | 0,009 | 6,08 * | 0,024 |
| PhP | 492,55± | 163,33± | 0,332± | 19,21± | 0,039± | 182,53± | 0,113± |
| | 4,85 | 7,84 * | 0,017 * | 6,35 | 0,013 | 12,16 * | 0,036 |

| | | | | | | | |
|---|---|---|---|---|---|---|---|
| *- obtained results are statistically significant (p<0.05, Dannet test) regarding the group exposed to the physiological solution. | | | | | | | |

The use of PhP in the course of the study has statistically significantly reduced the area of ischemia zone and the area of the total injury zone (ischemia zone plus necrosis zone). Anti-ischemic effect of PhP exceeded the one of Obsidan. It must be noted, that PhP administration nearly twice reduced the area of myocardial necrosis, though these data were not statistically significant. Hence, the substance has anti-ischemic effect, which exceeds the one of Obsidan.

Unlike activity of Obsidan, anti-ischemic activity of PhP is not followed by any negative inotrop effect. In the other study parameters of hemodynamics in rats exposed to PhP have been estimated (minute cardiac supply, cardiac index, cardiac output, heart rate). Hemodynamics parameters have been estimated by direct method on the fourth day after PhP administration in dose 50 mg/kg.

**Table 2.**

| Hemodynamics parameters in rats on the 4^{th} day after PhP administration | |
|---|---|
| Parameter | Value (% in comparison with source data before PhP administration) |
| Minute cardiac supply | 110.8 ±3.2 |
| Cardiac index | 133.8±9.8 |
| Cardiac output | 161.8±12.7 |
| Heart rate | 115.4±3.1 |

PhP has improved heart performance parameters at the expense of cardiac output increase. Consequently the substance obtains positive inotrop effect.

### Studies on general toxicity of PhP

Acute toxicity (LD₅₀) has been studied in rats, the substance being administered intraperitoneally and perorally. It has been shown that LD₅₀ of the substance, administered intraperitoneally is 538 mg/kg in males and 522.4 mg/kg in females. LD₅₀ of the substance administered perorally to rats (males and females) - 2464,8 mg/kg, of the pharmaceutical form - 3185,8 mg/kg.

In the course of the studies on PhP acute toxicity, PhP has been administered to rats and rabbits in dose 50 and 100 mg/kg; it was shown that PhP does not influence the parameters of the peripheral blood, composition of electrolytes and other biochemical parameters. The study on chronic toxicity in 160 rats and 28 dogs has demonstrated the absence of significant changes of peripheral blood and heart, kidney and liver functions in both animal types, exposed to PhP perorally in doses 50, 250 and 500 mg/kg.

The substance can be used in any suitable pharmaceutical form, containing effective quantity of PhP along with acceptable excipients.

Thus, the suggested remedy is of low toxicity and has anti-ischemic activity, exceeding the one of Obsidan. At the same time it does not have any negative inotrop activity. The fact of PhP's hypotensive and nootrop activity makes it a prospective remedy for the treatment of cardiovascular diseases.

### REFERENCE LITERATURE

1. Russian Federation Patent Nº 2054938, A 61 K 31/41, 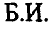 Nº6,1996.
2. Russian Federation Patent Nº 2058144, A 61 K 31/445, 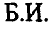 Nº11,1996.
3. Russian Federation Patent Nº1827067, A 61 K 31/41, 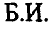 Nº19,1994.
4. M. D. Mashkovky. Medicinal remedies. Vol.1, Moscow, Medicine, 1993, page 330.
5. Inventor's Certificate USSR Nº 797219, C 07 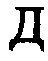 202/26, A 61 K 31/41. Bulletin of Inventions Nº21, 1995 r.
6. J.G. Bobkov, I.S.Morozov, O.M.Glozman et al. Pharmacological characteristic of piratsetam new phenyl analogue - 4- phenylpiratsetam.//Bulletin of experimental medicine and biology.-1983 r.,XCY, pages 50-53.

## Claims

1. Use of 4-phenyl-2-pyrrolidinone-1-acetic acid amide for the manufacture of a medicament for the treatment of myocardial ischemia.

## Patentansprüche

1. Verwendung von 4-Phenyl-2-Pyrrolidinon-1-Essigsäureamid zur Herstellung eines Arzneimittels für die Behandlung von Myocardialer Ischämie.

## Revendications

1. Utilisation de l'amide de 4-phényle-2-pyrrolidinone-1 acide acétique pour la production d'un médicament pour le traitement de l'ischemie myocardiale.
